# EUROPÄISCHE PATENTSCHRIFT

(11) **EP 0 999 849 B1**
(45) Veröffentlichungstag und Bekanntmachung des Hinweises auf die Patenterteilung: **04.09.2002**
(21) Anmeldenummer: 98928139.9
(22) Anmeldetag: 04.04.1998
(51) Int. Cl.: A61K 38/17, A61P 31/04

(54) **VERWENDUNG VON LBP ZUR SEPSISTHERAPIE**
USE OF LBP IN THE TREATMENT OF SEPTICAEMIA
UTILISATION DE LA PROTEINE LBP DANS LE TRAITEMENT DE LA SEPTICEMIE

(30) Priorität: 11.07.1997 DE 19729810
(43) Veröffentlichungstag der Anmeldung: 17.05.2000
(73) Patentinhaber: MAX-DELBRÜCK-CENTRUM FÜR MOLEKULARE MEDIZIN, 13125 Berlin (DE)
(72) Erfinder: SCHUMANN, Ralf, Reiner, D-16341 Zepernick (DE); LAMPING, Norbert, D-10435 Berlin (DE)
(74) Vertreter: Baumbach, Friedrich, Dr.
(86) Internationale Anmeldenummer: DE9800964
(87) Internationale Veröffentlichungsnummer: WO99002178

(56) Entgegenhaltungen:
- WO-A-94/25476
- WO-A-95/08560
- WO-A-95/25117
- GALLAY P ET AL: "Lipopolysaccharide - binding protein as a major plasma protein responsible for endotoxemic shock." PROCEEDINGS OF THE NATIONAL ACADEMY OF SCIENCES OF THE UNITED STATES OF AMERICA, (1993 NOV 1) 90 (21) 9935-8, XP002077616
- ABRAHAMSON S L ET AL: "Biochemical characterization of recombinant fusions of lipopolysaccharide binding protein and bactericidal/permeability- increasing protein. Implications in biological activity." JOURNAL OF BIOLOGICAL CHEMISTRY, (1997 JAN 24) 272 (4) 2149-55, XP002077617
- LAMPING N ET AL: "LPS-binding protein protects mice from septic shock caused by LPS or gram-negative bacteria." JOURNAL OF CLINICAL INVESTIGATION, (1998 MAY 15) 101 (10) 2065-71, XP002077618

## Beschreibung

Die Erfindung betrifft ein Mittel zur Sepsistherapie, seine Herstellung und seine Verwendung. Anwendungsgebiete der Erfindung sind die pharmazeutische Industrie und die Medizin.

Die Sepsis mit ihren häufig letalen Komplikationen gehört zu den am meisten gefürchtetsten und therapeutisch nicht beherrschbaren Krankheitsbildern in der Medizin und fordert jährlich mehrere 100.000 Todesopfer allein in den westlichen Ländern. Es muß dringend nach neuen Behandlungsmöglichkeiten gesucht werden, da Antibiotika zu langsam wirken und die Freisetzung bakterieller Toxine nicht verhindern, sie z.T. sogar verstärken. Die durch bakterielle Toxine ausgelöste Ausschüttung von Botenstoffen (Zytokine) durch den Wirtsorganismus ist das wichtigste Element der pathogenetischen Kaskade in der Entstehung des Krankheitsbildes der Sepsis. Verschiedene neue therapeutische Ansätze, zum Einen bakterielles Lipopolysaccharid (LPS) als wichtigstes Toxin durch Antikörper zu blockieren oder die körpereigenen, sogenannten proinflammatorischen Zytokine zu antagonisieren versagten in großen klinischen Studien vollständig (C. Natanson et al., Ann. Intern Med. 120, 771-783 (1994).

WO 95/08560 beschreibt den Einsatz von Peptiden mit dem Ziel, "die LPS-LBP Interaktion zu hemmen". Es handelt sich dabei um (kleine, von LBP abgeleitete) Peptide.

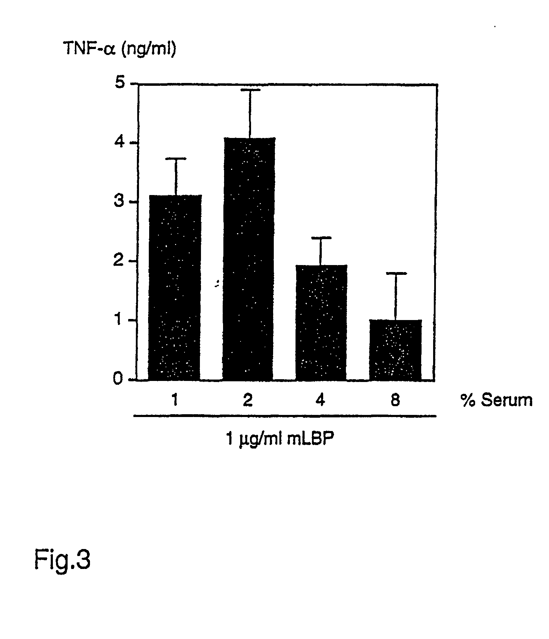

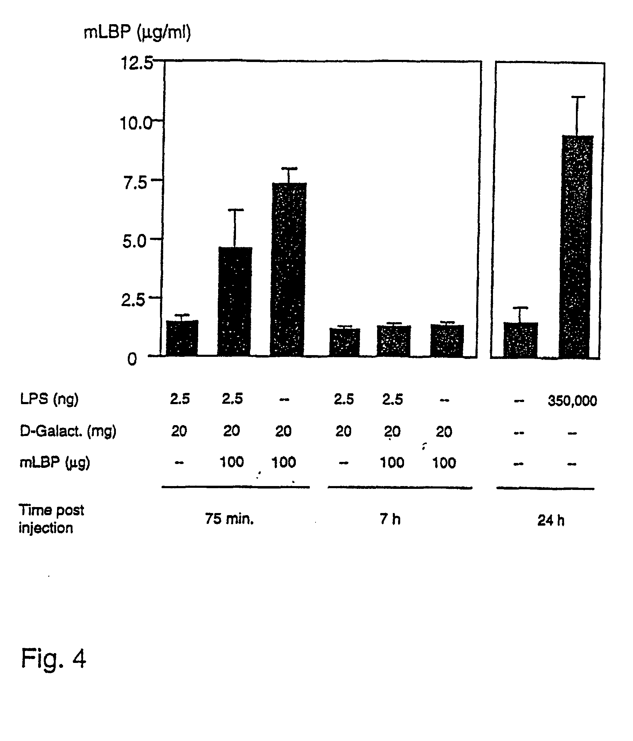

WO 98/25117 beschreibt (poly-)Peptide zur Hemmung der natürlichen (und von den Autoren als ungünstig für den Krankheitsverlauf angesehenen) Interaktionen von LBP mit LPS. Als Expressionsystem wurden CHO-Zellen für die Expression der Fragmente gewählt. Die erzielten Ergebnisse mit niedrig konzentriertem LBP führten zur Hemmung der natürlichen LBP-Aktivität. Die Abbildungen 3A, 3B und 4 zeigen sehr deutlich, wie das LBP-Fragment die natürlichen Funktionen von LBP inhibiert ja kompetiert.

WO 94/25476 hatte das Ziel, LPS-Effekte mittels BPI, modifiziertem LBP sowie mit Hilfe von BPI-LBP-Chimeren zu hemmen. Wie u.a. der Tabelle 1 entnommen werden kann, ist dargestellt, daß BPI LPS-Effekte hemmt, während LBP sie verstärkt. Es wird vorgeschlagen, LBP-Varianten zu entwickeln, die lediglich die LPS-Bindungskapazität besitzen und außerdem die (vorteilhaft) lange Halbwertzeit des LBP. Diese Halbwertszeit des LBP ist auch die Basis für die Herstellung von Chimeren, die die LPS-hemmende Wirkung von BPI ausnutzen soll.

Der Erfindung liegt die Aufgabe zugrunde, ein Mittel zur Sepsistherapie zu entwickeln.

Diese Aufgabe wird durch ein Mittel gelöst, das als wesentlichen Bestandteil das Lipopolysaccharid Bindende Protein LBP enthält.

Die Merkmale der Erfindung sind in den Ansprüchen 1-9 enthalten. Neben dem humanen LBP kann auch murines oder Kaninchen-LBP eingesetzt werden.

Die Struktur von LBP ist bekannt, seine Gewinnung erfolgt durch Isolierung eines Klons aus einer Akutphase cDNA Genbank, sowie anschließender Sequenzierung und Ableitung der Aminosäuresequenz. Rekombinantes LBP wird durch Klonierung der cDNA in einen Expressionsvektor sowie Ko-Infektion von Insektenzellen mit dem Baculovirus hergestellt.

Das klonierte LPS Bindende Protein (LBP) bindet hochaffin LPS und wird während der Sepsis als Akutphaseprotein ins Serum ausgeschüttet. Wie in den Ausführungsbeispielen gezeigt wird, hemmt LBP die LPS-Wirkungen und kann, wenn es Mäusen gegeben wird, die durch LPS ausgelöste Sepsis unterdrücken und die Letalität hochsignifikant reduzieren. Dies gilt bei Einsatz gleichzeitig mit der Auslösung der Sepsis, sowie vorher, was LBP für eine Sepsisprophylaxe geeignet erscheinen läßt. Da LPS auch bei der durch grampositive Bakterien ausgelösten Sepsis, sowie bei dem 'systemic inflammatory response syndrome', einem durch Trauma ausgelösten, der Sepsis identischen Krankheitsbild, durch 'Translokation' gramnegativer Darmbakterien eine zentrale Rolle spielt, kann die Hemmung der LPS-Effekte durch LBP auch diese dramatischen Krankheitsbilder verbessern.

Eine weitere grundlegende Möglichkeit zur Realisierung der Erfindung besteht darin, das klonierte LBP-Gen in einen adenoviralen Vektor mit hoher Leberaktivität hinter den starken CMV-Promoter zu klonieren, um so einen Gentransfer nebst hepatischer Expression von LBP zu erreichen.

Die Erfindung ist anwendbar bei
- Sepsis ausgelöst durch gramnegative Bakterien
- Sepsis ausgelöst durch grampositive Bakterien
- Systemic inflammatory response syndrom (SIRS), ausgelöst durch Trauma und Verletzung

Die Erfindung soll nachfolgend durch ein Ausführungsbeispiel und Abbildungen näher erläutert werden.

### Die Gewinnung von LBP

Die komplette LBP cDNA wird in den pACHLT-B Vektor (Pharmingen, San Diego, USA) hinter den starken Polyhedrinpromoter und hinter die Glutathion S-Transferase (GST) cDNA kloniert, so daß ein GST-Fusionsprotein exprimiert wird. Es wird dann eine 500 ml Zellkultur von Sf-9-Insektenzellen mit diesem Vektor und der Baculovirus DNA ('Baculogold', Baculovirus DNA in linearisierter Form, ebenfalls von Pharmingen, San Diego, USA) infiziert. Die Zellen werden nach 2 Tagen lysiert und das Lysat wird im 'batch'-Verfahren an Gluthation-Sepharose in Gegenwart von Triton X-100 gekoppelt. Durch Thrombinverdau wird dann LBP vom Fusionspartner abgespalten, gefolgt von einer Behandlung mit Calbiosorb zur Triton-Entfernung und einer Behandlung mit Benzamidin-Sepharose zur Entfernung von Thrombinresten. Die resultierenden Konzentrationen von reinem LBP betragen 0,3 - 0,5 mg/ml.

### Legenden zu den Abbildungen

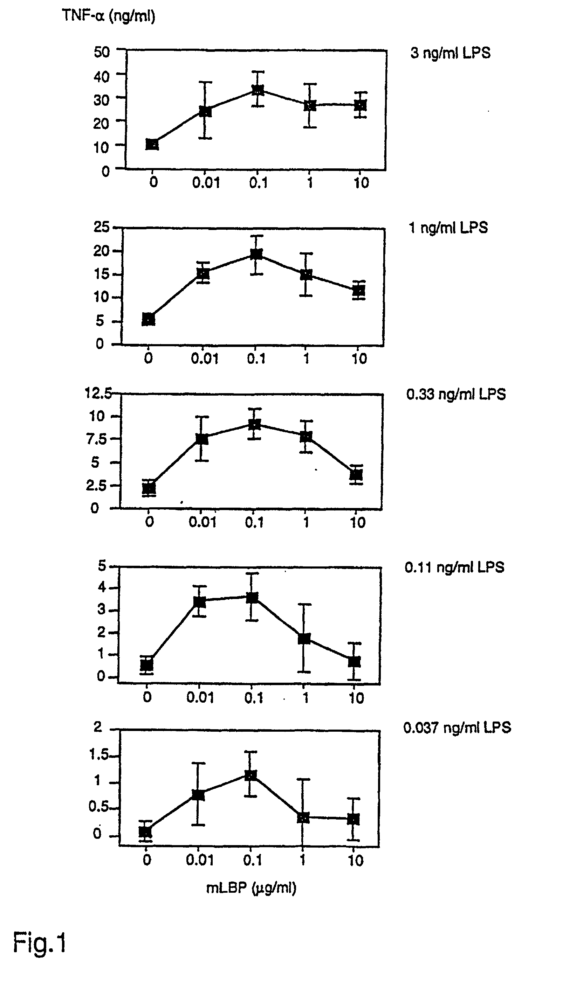

Abbildung 1: Eine murine Makrophagenzellinie wird mit verschiedenen Konzentrationen des bakteriellen Toxins LPS in vitro zur Synthese des Sepsismediators TNF in Abhängigkeit von LBP stimuliert. Bei hohen, im Organismus nicht vorkommenden LPS-Konzentrationen beeinflusst LBP die TNF-Synthese nicht. Die Stimulation der Makrophagen durch niedrigere LPS-Mengen wird jedoch durch hohe LBP-Kozentrationen, wie sie während der Akutphase in vivo vorkommen und auch durch exogene Zugabe erzielt werden können, gehemmt.

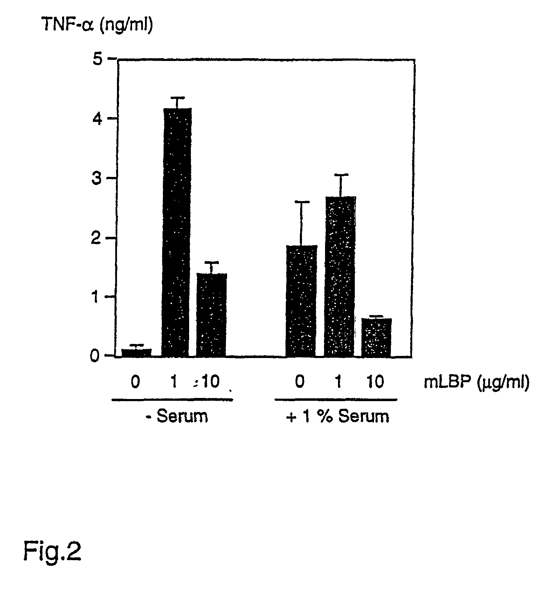

Abbildung 2: Hier zeigt sich, daß die Zugabe von LBP in der Gegenwart von Serum ebenfalls die TNF-Produktion der Makrophagenzellinie unterdrückt. Hierfür ist das im Serum befindliche LBP verantwortlich.

Abbildung 3: Wird die Serumkonzentration bei konstanter exogen zugeführter LBP-Menge erhöht, wird ebenfalls die TNF-Synthese unterdrückt.

Abbildung 4: Hier wird gezeigt, daß die LBP-Spiegel der Maus, die durch exogene Gabe erzeugt werden, den Akutphasespiegeln, die durch hohe LPS-Gabe erzeugt werden, entsprechen und somit physiologisch sind.

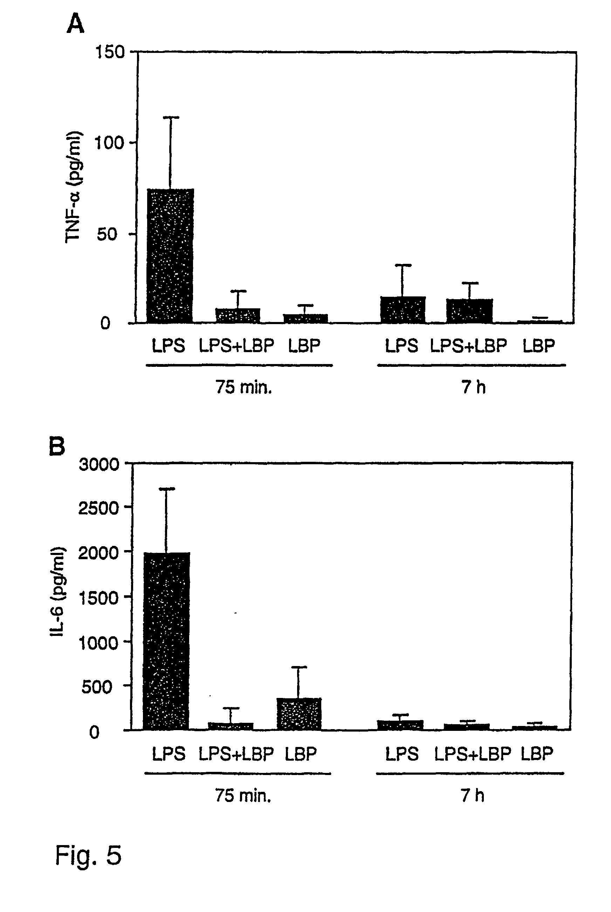

Abbildung 5: In der Maus kann durch gleichzeitige Gabe von LBP die LPS-induzierte Zytokinausschüttung unterdrückt werden. A: TNF, B: IL-6.

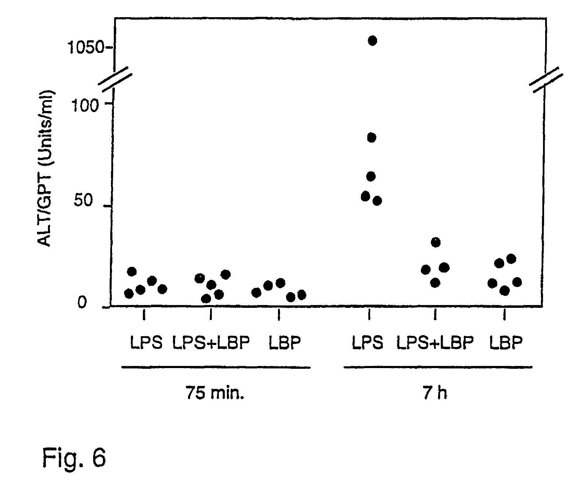

Abbildung 6: Desweiteren wird der durch LPS ausgelöste und durch den Anstieg des ALT-Enzym erfaßte Leberschaden durch die gleichzeitige Gabe von LBP unterdrückt.

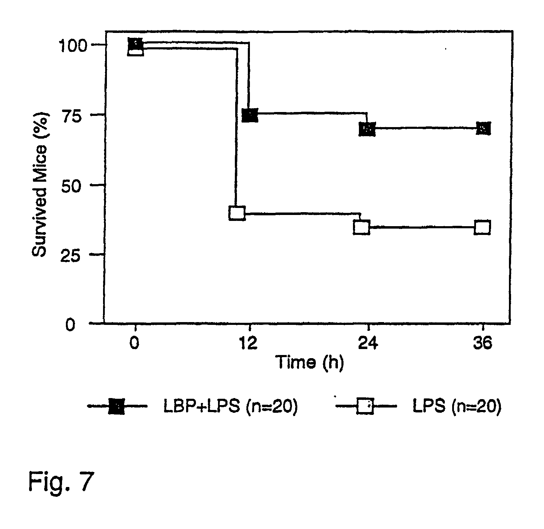

Abbildung 7: Die Gabe von LBP reduziert signifikant die Letalität in einem LPS-Sepsismodell, durchgeführt mit 20 Mäusen pro Gruppe.

### Kurze Interpretation:

Hochdosiertes LBP unterdrückt in vitro die durch das bakterielle Toxin LPS ausgelöste Synthese eines wichtigen Sepsismediatormoleküls, nämlich TNF. Die Produktion dieses Proteins und anderer Mediatoren wird in der Maus bei gleichzeitiger LBP-Gabe ebenfalls unterdrückt. Desweiteren wird der durch die LPS-Gabe induzierte Leberschaden durch LBP verhindert und die Überlebenszahl der Mäuse signifikant erhöht. Die Gabe von LBP scheint also vor den LPS-Effekten in der Sepsis zu schützen und stellt somit ein neues therapeutisches Prinzip zur Behandlung der Sepsis dar.

## Patentansprüche

1. Verwendung von dem Lipopolysaccharid Bindenden Protein (LBP) zur Herstellung eines Arzneimittels zur Hemmung von Lipopolysaccharid (LPS) zur Sepsistherapie.

2. Verwendung nach Anspruch 1, **dadurch gekennzeichnet, dass** humanes LBP verwendet wird.

3. Verwendung nach Anspruch 1, **dadurch gekennzeichnet, dass** murines LBP verwendet wird.

4. Verwendung nach Anspruch 1, **dadurch gekennzeichnet, dass** Kaninchen- oder Ratten-LBP verwendet wird.

5. Verwendung nach einem der Ansprüche 1 bis 4, **dadurch gekennzeichnet, dass** das Arzneimittel zur Therapie der durch gramnegative Bakterien ausgelösten Sepsis verwendet wird.

6. Verwendung nach einem der Ansprüche 1 bis 4, **dadurch gekennzeichnet, dass** das Arzneimittel zur Therapie der durch grampositive Bakterien ausgelösten Sepsis verwendet wird.

7. Verwendung nach einem der Ansprüche 1 bis 4, **dadurch gekennzeichnet, dass** das Arzneimittel zur Therapie des durch Trauma und Verletzung ausgelösten Systemic Inflammatory Response Syndroms (SIRS) verwendet wird.

8. Verfahren zur Herstellung eines Arzneimittels zum Gentransfer in die Leberzellen, **dadurch gekennzeichnet, dass** das LBP-Gen in einen adenoviralen Vektor hinter einen starken Promoter, vorzugsweise den CMV-Promoter, kloniert wird.

9. Verfahren zur Herstellung von LBP nach einem der Ansprüche 1 bis 4, **dadurch gekennzeichnet, daß** im Baculovirussystem, sowie in CHO-Zellen LBP zunächst als Fusionsprotein exprimiert und dann durch den Fusionspartner mittels an sich bekannter Trennverfahren aufgereinigt wird.

## Claims

1. Use of the lipopolysaccharide binding protein (LBP) to manufacture a medication to inhibit lipopolysaccharide (LPS) for sepsis therapy.

2. Use according to Claim 1, wherein human LBP is used.

3. Use according to Claim 1, wherein murine LBP is used.

4. Use according to Claim 1, wherein rabbits' or rats' LBP is used.

5. Use according to one of the Claims 1 to 4, wherein the medication is used for the therapy of the sepsis caused by gram-negative bacteria.

6. Use according to one of the Claims 1 to 4, wherein the medication is used for the therapy of the sepsis caused by gram-positive bacteria.

7. Use according to one of the Claims 1 to 4, wherein the medication is used for the therapy of the Systemic Inflammatory Response Syndrome (SIRS) caused by trauma and injury.

8. Method for the production of a medication for the gene transfer into the hepatic cells, wherein the LBP gene is cloned into an adenoviral vector behind a strong promoter, preferably the CMV promoter.

9. Method for the production of LBP according to one of the Claims 1 to 4, wherein LBP is firstly expressed as a fusion protein in the baculovirus system as well as in CHO cells and then cleansed by the fusion partner by means of separation processes known per se.

## Revendications

1. Emploi de la protéine Lipopolysaccharide Binding Protein (LBP) pour la fabrication d'un médicament destiné à inhiber le lipopolysaccharide (LPS) pour le traitement de la septicémie.

2. Emploi selon la revendication 1, se caractérisant par le fait que l'on utilise la LBP humaine.

3. Emploi selon la revendication 1, se caractérisant par le fait que l'on utilise la LBP murine.

4. Emploi selon la revendication 1, se caractérisant par le fait que l'on utilise la LBP de lapins ou de rats.

5. Emploi selon l'une des revendications 1 à 4, se caractérisant par le fait que l'on utilise le médicament pour le traitement de la septicémie déclenchée par des bactéries Gram -.

6. Emploi selon l'une des revendications 1 à 4, se caractérisant par le fait que l'on utilise le médicament pour le traitement de la septicémie déclenchée par des bactéries Gram +.

7. Emploi selon l'une des revendications 1 à 4, se caractérisant par le fait que l'on utilise le médicament pour le traitement du Syndrome Systemic Inflammatory Response (SIRS) déclenché par un traumatisme et par une blessure.

8. Procédé de fabrication d'un médicament pour le transfert génétique dans les cellules du foie, se caractérisant par le fait que le gène LBP est cloné en vecteur adénoviral derrière un promoteur puissant, de préférence le promoteur CMV (cytomégalovirus).

9. Procédé de fabrication de la LBP selon l'une des revendications 1 à 4, se caractérisant par le fait que dans le système baculovirus, ainsi que dans les cellules CHO, la LBP sera tout d'abord exprimée en tant que protéine de fusion puis purifiée par le partenaire de fusion au moyen d'un processus de séparation connu en soi.
